# EUROPEAN PATENT SPECIFICATION

(11) **EP 1 786 603 B1**
(45) Date of publication and mention of the grant of the patent: **16.09.2009**
(21) Application number: 05769510.8
(22) Date of filing: 28.07.2005
(51) Int. Cl.: B26B 13/26, A01G 3/02, B26B 13/28, A61B 17/32, B26D 3/00, B26D 3/16, B23D 29/02, B26B 17/02

(54) **SCISSORS WITH SLIDE CUTTING**
SCHERE FÜR GLEITENDEN SCHNITT
CISEAUX AVEC DÉCOUPE LATÉRALE

(30) Priority: 06.08.2004 IT FI20040178
(43) Date of publication of application: 23.05.2007
(73) Proprietor: Tellini, Luigi, I-52100 Arezzo (IT)
(72) Inventor: Tellini, Luigi, I-52100 Arezzo (IT)
(74) Representative: Bardini, Marco Luigi
(86) International application number: PCT/IT2005/000452
(87) International publication number: WO 2006/013606

(56) References cited:
- EP-A- 0 074 578
- DE-C- 323 923
- FR-A- 2 532 235
- GB-A- 256 728
- GB-A- 819 545
- US-A- 2 495 677
- US-A1- 2002 124 413
- PATENT ABSTRACTS OF JAPAN vol. 2000, no. 15, 6 April 2001 (2001-04-06) & JP 2000 342866 A (MUROMOTO TEKKO KK), 12 December 2000 (2000-12-12)
- PATENT ABSTRACTS OF JAPAN vol. 2003, no. 01, 14 January 2003 (2003-01-14) & JP 2002 253877 A (FISKARS CONSUMER OY AB), 10 September 2002 (2002-09-10)

## Description

### Field of the invention

The present invention relates to the field of cutting devices, in particular that of scissors. More specifically it relates to a new type of scissors with slide cutting, intended in particular, albeit not exclusively, for uses such as the pruning of plants, the cutting of organic tissue in surgery and of objects with a tubular shape and/or with low compression resistance.

### Description of the prior art

With reference to scissors, shears or similar devices, the most traditional cutting operation involves a simple rotation of the two blades around a central pin. Consequently, two points which are equidistant from the central pin on the edge of the respective blades, following the cutting action merely move closer to each other along a circular path, centered on said pin. The term "blades", here and further on in the present description, refers to the two jaws which seize the object to be cut and which are not necessarily cutting and sharp. In fact, in many types of scissors one of the two blades is configured as an anvil, functioning as a stop for the object to be cut, and as an abutment for the actual cutting blade.

A different kinematic cutting mechanism, the so-called "slide" type, although to date less used and recognized, in theory offers many significant advantages compared to the previous one. Considering the cutting axis - or cutting line - defined by the line along which cutting is completed (a line which, in traditional symmetrical scissors, coincides with the axis of symmetry of the assembly of the two blades, passing through the rotation pin of the same), in slide cutting the two blades, in addition to mutually approaching due to a rotational movement, also perform a movement of mutual translation parallel to the cutting line. In practice, considering two points on the edge of respective blades which match when the blades are closed, the perpendicular projections of said points on the cutting line become mutually displaced along this line due to the opening movement of the scissors.

With slide cutting, the object to be cut is incised layer after layer, so that the blade moves gradually, requiring a slighter pressure. Cutting takes place slowly, with improved possibility of control, and with a highly accurate result. The advantages mentioned above, compared to traditional cutting, can therefore be summarized in the following terms: light pressure exerted during cutting, without deformation and with minimum damage to the cut object, greater precision and cleanliness of the cut; cutting is less prone to jamming; the annoying clicking noise at the end of cutting is minimized; the muscular force required for performing cutting is less intense.

Clearly, in order to be carried out, the slide cutting requires changes to be made to the conventional structure of standard scissors. The prior art provides various examples of scissors designed for this purpose. Basing on the conventional structure, the simplest change for obtaining the slide action is to merely off-center the rotation pin of the two blades with respect to the cutting line. The latter becomes defined with good approximation by the inside edge of the anvil, or of a semi-cutting blade. In order to obtain a significant sliding, and therefore with satisfactory effectiveness, the distance between the rotation pin and cutting line must however be considerable, and this has a negative effect on the ergonomics and ease of actuation. A device of this kind is described in PCT international patent publication no. WO8804223. For obtaining even a small angle of aperture of the blades, the handles (that is to say the grip portions integral with the respective blades, on the opposite side in relation to the rotation pin) must be considerably displaced apart, so that actuation is extremely difficult, even more so with one single hand. Moreover, the Z-shape of the device implies that, with the blades open, there is a markedly asymmetrical configuration. Finally, during actuation, the device tends to rotate over the cutting plane.

Other attempts have been made in different directions. In some cases use has been made of one or more slide guides (for example with pin and slot systems, as in US patents nos. 2837823 and 2528816). However, this solution entails a sliding of small extent, .otherwise the scissors become usable with extreme difficulty. Moreover, the sliding guide is strongly exposed to jamming, particularly considering that scissors are often used in conditions of humidity, dust or in potential contact with actual waste.

A certain number of known-solutions are also based on systems wherein the two blades are pivotally connected by a pair of levers so as to realize an articulated parallelogram kinematic mechanism. The scissors which are disclose in US patents nos. 4525929 and 4677747 relate in particular to this type. In this case the two blades always remain parallel, and the handles are actuated with a movement which is much more inconvenient compared to that of simple closure, typical of standard scissors. The ergonomics of the device is even worse if a significant sliding is to be obtained, making actuation with one single hand impossible.

Finally a solution is known which provides, in addition to the previously mentioned off-centering of the rotation pin of the two blades in relation to the cutting line, a simultaneous fracture of one of the two blades in relation to the relative handle, as in US patent no. 2495677. In practice, while one of the blades, which can be defined as "stationary" blade, forms a single integral body with the relative handle, the other blade is "movable" in relation to its own handle, and articulated thereto via a pivotal connection. This latter connection is located near the edge of the movable blade, while the pivotal connection between the two blades is defined by a pin near the stationary blade.

The trajectory of the movable blade is guided by a sliding abutment on the handle of the stationary blade, which already constitutes a limit in that, as mentioned previously, the risk of malfunctioning and/or jamming is not negligible. Moreover, due to the sliding guide system, a cutting action takes places which tends to displace the axis of the object to be cut, and is therefore inconvenient and awkward. Moreover, mutual sliding of the two blades is reduced in extent, and above all occurs in an unfavorable direction. Imagining that the stationary blade is kept still, as normally happens when using the device, the movable blade slides forwards, i.e. from the grip side towards the front. This directionality of movement is disadvantageous, in that it causes an extension of the device outwards, with consequent risk of meshing of the movable blade or of accidental and undesired cuts. This is even more relevant if we consider that the area of extension is the furthest one, less visible and with less control by the user, particular when bushes have to be pruned or in other situations wherein the cutting area is not open and clear.

### Summary of the invention

In conclusion, the prior art lacks of a slide cutting scissors device with the following characteristics: ease and ergonomics of use, in particular with one single hand and maintaining a cutting action comparable to the familiar and established cutting action of traditional scissors; sliding of significant extent (not less than one centimeter) and in the most favorable direction (that is to say, front to rear); efficiency and reliability of operation, minimizing the risk of jamming; structural simplicity; universality of use.

The present invention achieves the object of combining all the requirements listed above, providing scissors with slide cutting the essential features of which are defined by the first of the annexed claims.

### Brief description of the drawings

The characteristics and advantages of the scissors with slide cutting according to the present invention will be made clearer by the following description of its embodiments, given purely as a an example and not limitative, with reference to the accompanying drawings, wherein:
- figures 1 and 2 show schematically, in a side view, a first embodiment of the scissors according to the invention, respectively in the opening and closure configuration;
- figure 3 represents schematically, again in a side view, a second embodiment of the scissors according to the invention, in the open configuration;
- figures 4 and 5 show schematically, in a side view, a third embodiment of the scissors according to the invention, respectively in the opening and closure configuration;
- figures 6 to 8 are side views of scissors according to a fourth embodiment of the invention, respectively in the open, partially closed and fully closed configuration; and
- figures 9 and 10 show schematically, in a side plane view, the scissors according to the invention in a fifth embodiment, respectively in the opening and closure configuration.

### Description of preferred embodiments

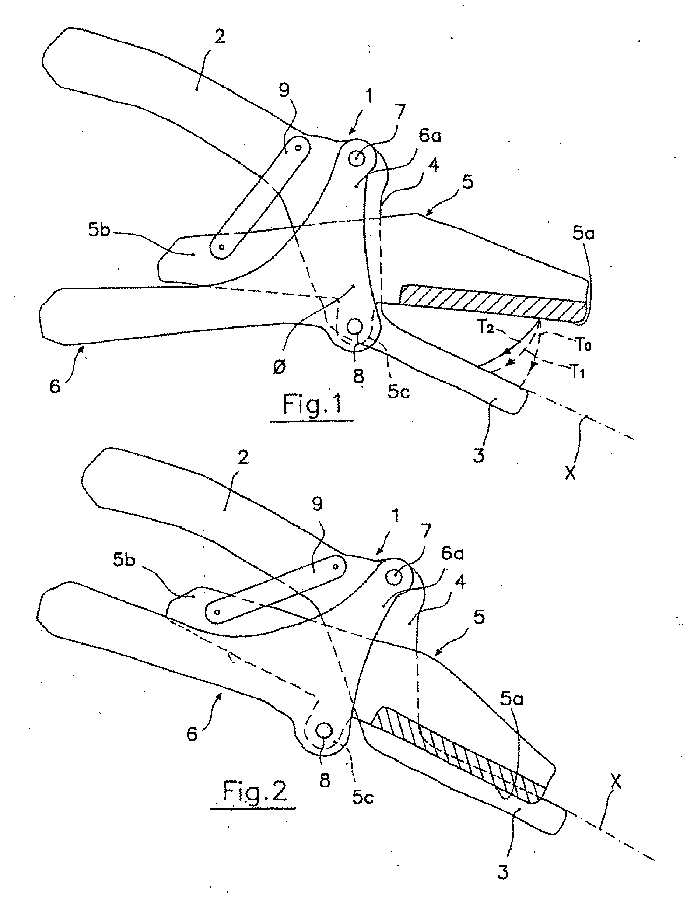

Referring to figures 1 and 2, there is shown a pair of scissors according to the invention, of the so-called anvil type, i.e. with only one cutting blade and the other, shaped in fact as an anvil, with an abutment and stop function. Aside from this example, a similar overall configuration may clearly be adopted with both blades having cutting or semi-cutting functions, and with or without overlapping of the blades.

The scissors comprise a substantially S-shaped stem 1, defining a handle 2 and a blade 3. The blade 3 in this case, as mentioned, is shaped and works as an anvil, and is integral with the relative handle 2, being therefore stationary in relation thereto. The stationary blade 3 and the handle 2, substantially parallel, are joined by a central diagonal portion 4, transverse in relation to a cutting line X, substantially defined by the inner edge of the stationary blade 3.

The scissors also comprise a second blade 5 and a relative handle 6 which, contrary to the stationary blade 3 and the handle 2, are not integral with each other to form an entire stem, so that they can be defined respectively as "movable blade" 5 and "truncated handle" 6. In greater detail, the truncated handle 6 is substantially L-shapes, with a foot 6a protruding transversally, for a length that is substantially equal to that of the central diagonal portion 4 of the stem 1. The foot 6a is hinged at its free end to the central diagonal portion 4 by means of a first rotation pin 7, placed at the root of the handle 2 of the stationary blade 3.

The movable blade 5 is pivotally connected at the other end of the foot 6a, i.e. in practice at the turn of the L defined by the truncated handle 6, turn which is located near the root of the stationary blade 3. The movable blade 5, with a cutting edge 5a which can partially overlap the stationary blade 3, has a tail 5b which projects towards the truncated handle 6, beyond the relative foot 6a. In a central position, i.e. between the cutting edge 5a and the tail 5b, a nosepiece 5c projects transversely from the movable blade 5. The truncated handle 6 is pivotally connected to the nosepiece 5c via a second rotation pin 8.

Finally, a control lever 9 extends between the tail 5b of the movable blade 5 and the root of the handle 2 of the stem 1. The lever 9 is pivotally supported at both ends, and has in its turn an oblique transverse arrangement, in this case oppositely angled with respect to that of the central diagonal portion 4 of the stem 1.

As one can clearly appreciate by comparing the two drawings, the cutting movement of the scissors takes place in the following manner. Starting with the closed configuration of figure 2, the moving apart of the handles 2 and 6 entails the rotation of the truncated handle 6 around the first pin 7. Simultaneously the movable blade 5, in addition to rotating away from the stationary blade 3 along with the truncated handle 6 around the aforesaid first pin 7, being constrained and controlled by the lever 9 also performs a certain relative rotation in relation to the truncated handle 6 around the second pin 8, the relative rotation around the second pin 8 being in an opposite direction to the absolute rotation around the first pin 7.

Having reached the open configuration of figure 1, the cutting action will correspond to the handles 2 and 6 moving close to each other, according to the standard mode of operation of traditional scissors. The movements already described will be performed in an opposite direction. The movable blade 5 will rotate and translate towards the stationary blade 3, with a movement which, in this embodiment, is the combination of an absolute rotation around the first pin 7 and a relative rotation around the second pin 8 controlled by the lever 9. The translatory component of the motion, directed from the front side of the device to the side of the grip, is that which determines relative sliding of the two blades, indicated by the paths shown in figure 1.

In particular, given a theoretical path T0 without sliding (conventional scissors with rotation pin in a fulcrum point Ø on the imaginary axis of symmetry of the blades), and a further theoretical path T1 with a certain sliding which can be obtained simply through the off-centering of the first pin 7 in relation to the cutting line X (envisaging the movable blade 5 integral with the relative truncated handle 6), the real path T2 which occurs with the scissors according to this embodiment of the invention involves a further significant sliding, as a result of the possibility of relative rotation of the movable blade 5 in relation to the truncated handle 6. This relative rotation, controlled by the lever 9, in practice delays the approaching of the movable blade 5 towards the stationary blade 3, making the path more linear and enabling further sliding backwards both of the blade 5 and of the relative handle 6. There occurs an overall sliding of considerable extent and, at the same time, a very convenient and easy mode of operation for the user.

The functional concept described above (absolute rotation of a movable blade in relation to the stationary blade around a fulcrum which is off-centered from the cutting line on the side of the movable blade itself, and simultaneous relative rotation of the movable blade in relation to its truncated handle controlled by control means articulated between the movable blade and the stem of the stationary blade), can be applied in various other embodiments, described hereafter by way of example.

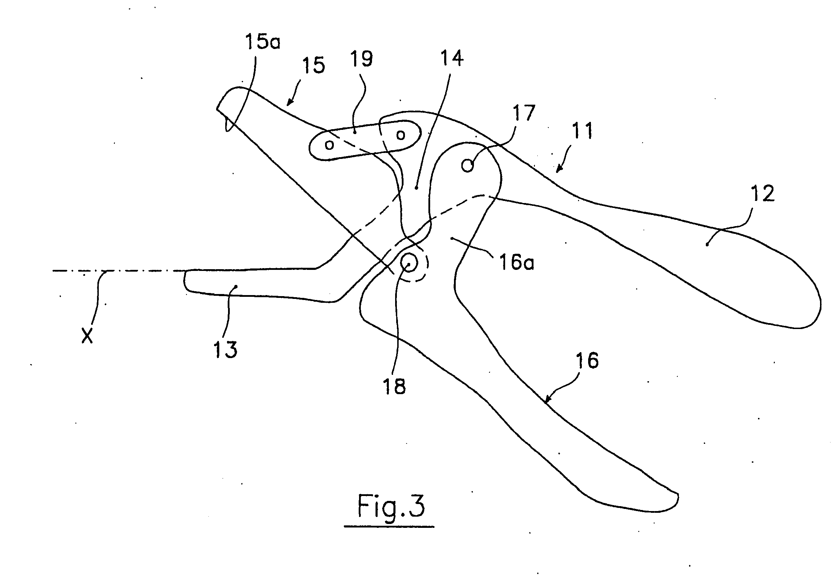

Referring first of all to figure 3, wherein parts identical or corresponding to those already mentioned for the first embodiment have corresponding reference numerals and are not described further, it can be noticed that the movable blade 15 is in this case lacking a tail extending in a rearward direction beyond the attachment to the truncated handle 16 (that is to say, beyond the second rotation pin 18). In fact, the lever 19, instead of being located at the rear, connected to said tail, is in a frontal position, between two points of articulation, respectively on the central diagonal portion 14 of the stem 11 ahead of the first rotation pin 17, and on the movable blade 15. The lever 19 is therefore arranged diagonally with similar slant in relation to that of the central diagonal portion 14.

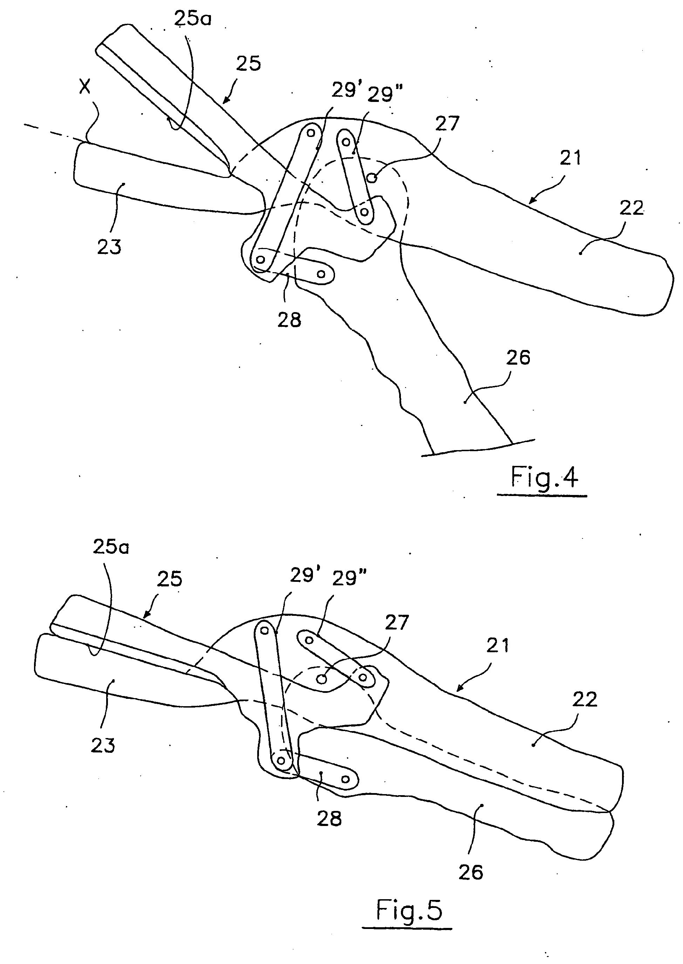

A third embodiment is illustrated in figures 4 and 5, to which reference is made hereafter. In this case, the first rotation pin 27 is off-centered to a lesser extent, if compared to the previous embodiments. However, a slightly more complex construction is required, with two control levers 29', 29" between the movable blade 25 and the intermediate diagonal portion 24 of the stem 21, Furthermore, the movable blade 25 and the relative truncated handle 26 are connected indirectly via an additional articulation arm 28, replacing the simple rotation pin of the previous examples.

As clearly emerges from the comparison between figures 4 and 5, the cutting action is in any case similar to that already seen. The movable blade 25 performs an absolute rotation, along with the truncated handle 26, around the first rotation pin 27. Simultaneously, thanks to the levers 29', 29", the movable blade 25 is articulated with respect to the truncated handle 26, with a motion which in this case, in addition to a relative rotatory component, comprises an actual translatory component, as a result of the indirect connection via the arm 28.

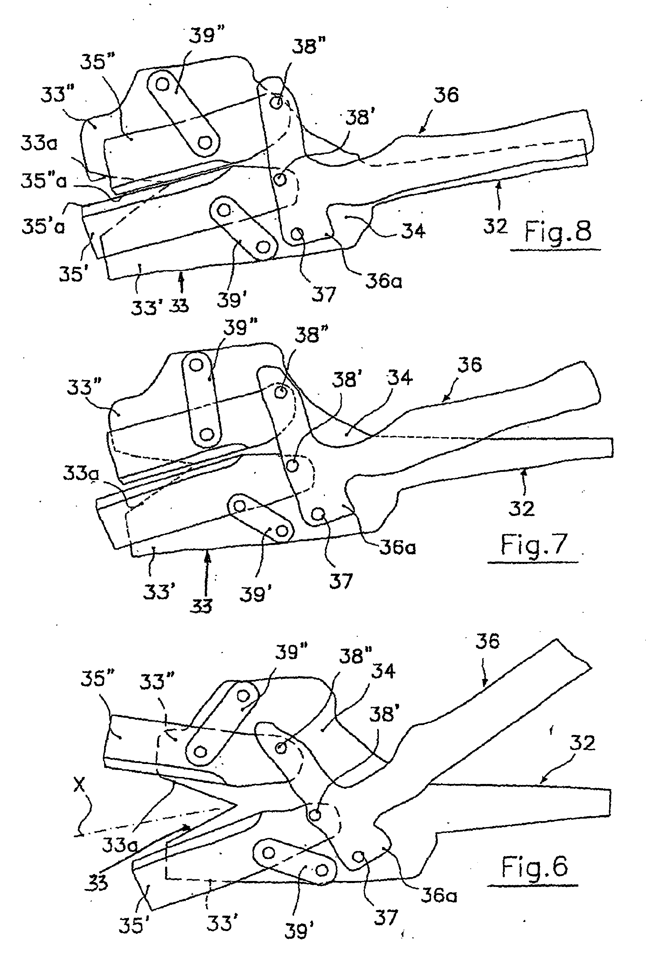

Referring now to figures 6 to 8, in a further embodiment of the invention there is a doubling of the basic configuration proposed in the first embodiment, with a truncated handle 36 which, instead of holding only a single movable blade, holds a pair of them, denoted as 35' and 35", mutually closing with a certain sliding along the cutting line X. The cutting line X is defined in this case by the axis of symmetry of the pair of movable blades. The foot 36a of the truncated handle 36 therefore provides, besides to the attachment on the root of the stationary handle 32 via the first rotation pin 37, a couple of second rotation pins 38' and 38", each for a relative movable blade 35' and 35".

The stationary blade consists in turn of a double structure, with two arms 33' and 33" extending frontally from a central connection portion 34 integral with the stationary handle 32. A V-shaped cavity 33a, substantially symmetrical in relation to the cutting line X, is defined between the two arms 33' and 33" , with a function of support and abutment of the object to be cut. The relative rotation of each movable blade 35', 35" around its own second rotation pin 38', 38" is controlled by respective levers 39'; 39", each articulated between a movable blade. 35', 35" and one of the arms 33', 33" of the stationary blade. Similarly to the second embodiment (figure 3), here too the points of attachment of the control levers 39' and 39" to the movable blades 35' and 35" are in a frontal position, i.e. closer to the tip of the blades than the points of attachment of the same blades to the truncated handle 36.

Cutting again takes place via the same procedure described above. An interesting special feature of this embodiment, shared with the second embodiment of figure 3, consists of the fact that the movable blades have a substantially swinging cutting movement. A first phase (figure 6) wherein the rotatory component leads to a mutual approaching of the movable blades (or of the movable blade towards the stationary blade in the case of figure 3) is followed by a phase wherein the blades almost come into tangential contact (figure 7) with a movement which, for a short extent, is substantially parallel to the cutting line X.

The continuation of the relative rotatory movement (figure 8) can finally, correspond to a phase of slight moving away of the blades, and of displacement of the cutting line towards the blade 35" . In practice, a portion of the object already involved in the cutting action of the blade 35" is at this point also reached by the movement of the blade 35'. This final phase is advantageous in that it can ensure complete cutting and prevent the jamming which may occur at the end of the same. More generally, the development of the cutting motion just described ensures the achievement of a sliding of a particular extent.

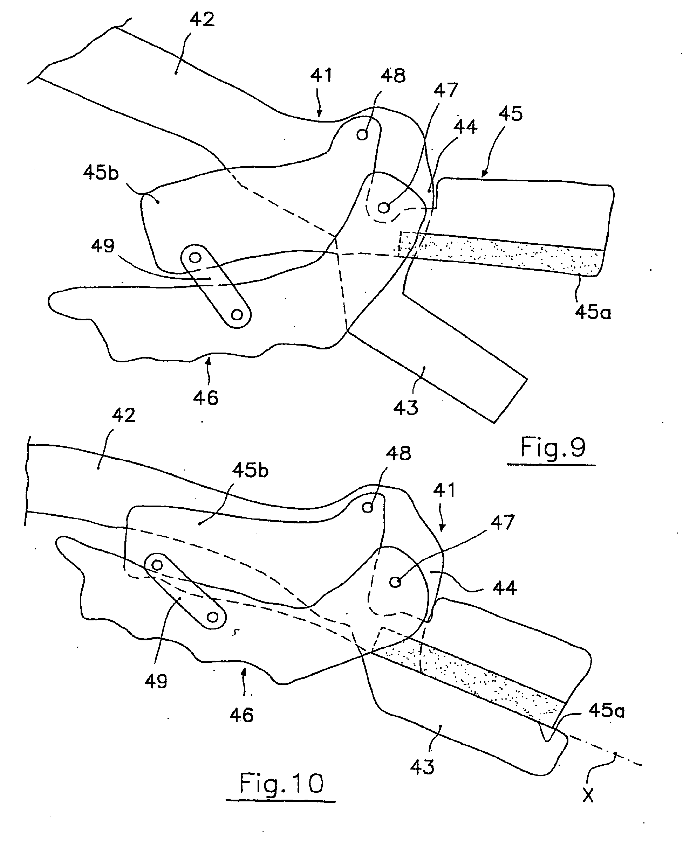

Referring finally to figures 9 and 10, in a fifth embodiment the invention has a construction which is different from the previous ones, yet in any case potentially advantageous compared to the prior art. In this case, the movable blade is pivotally connected directly to the stationary blade. However, the following features are in common with the previous embodiments of the invention: a movable blade, fractured with respect to the relative (truncated) handle; a truncated handle hinged to the stationary blade via a first rotation pin off-centered from the cutting line on the side of the movable blade; a movable blade supported in at least two points via purely pivotal connections, at least one of said points representing the connection point between the movable blade and control means which control the movement of the same blade in response to the mutual closure between the two handles.

In this case too there is an overall configuration which is new when compared to known solutions. This configuration is also advantageous, as far as the ergonomics of: actuation is concerned, in that the sliding between the two blades, which always takes place in the most favorable direction, is obtained with a small mutual rotation of the two handles, which can be actuated with one single hand and with an ergonomic and natural movement.

In detail, there can be noticed a S-shaped stem 41 with stationary handle 42 and stationary blade 43 tied by a central diagonal portion 44. The truncated handle 46 is pivotally connected to the central diagonal portion 44 via a first rotation pin 47 again in a position off-centered in relation to the cutting line X. The second rotation pin 48 now pivotally connects the movable blade 45 not to the truncated handle but to the stem 41, at the central portion 44, in a more peripheral position compared to the first rotation pin 47. Finally, a control lever 49 extends between the tail 45b of the movable blade 45 and the truncated handle 46. The lever 49 is pivotally supported at both ends.

The comparison between figures 9 and 10 shows the working with which the scissors cut. The movable blade 45 approaches to the stationary blade 43 with a movement whose translatory component (that, which gives rise to slide cutting) is due simply to the off-centering of the relative rotation pin 48 in relation to the cutting line X. Considering however the movement of the movable blade 45 with respect to the truncated handle 46, it is clear that, in practice, these two parts are articulated to each other in the manner of an articulated parallelogram, through the control lever 49 and the central portion 44 of the stem 41. In this case too there is a combination of movements, although with different directionalities compared to the previous embodiments. The second rotation pin 48, in turn markedly off-centered, is not fixed in relation to the first pin 47, and translates backwards during the closure operation. Therefore, by keeping the truncated handle 46 still, the stationary blade 43 moves slightly forwards with a purely rotatory movement, whereas the movable blade moves backwards with an essentially translatory movement. Consequently, there is an advantageous effect of "scaling down", that is to say, the mutual rotation (with sliding) between movable blade and stationary blade is obtained with a movement between truncated handle and, stationary handle having a reduced extent and a favorable orientation.

It is clear from the above how the scissors according to the invention represent a significant step forwards compared to all the solutions provided to date by the prior art. Slide cutting of real effectiveness is obtained, in terms of extent and directionality (towards the rear, or grip side), with purely pivotal connections, i.e. without sliding guide systems. Above all, the device can maintain an overall configuration and operating mode which are wholly similar to those, familiar to all users, of traditional scissors. The moving apart of the handles, required for actuation, is relatively small and in any case substantially symmetrical, so that the device is easy to handle and convenient to actuate even with one single hand. This leads to a virtually total universality of use, ranging from common domestic uses to more specialist uses such as in botany, agriculture and horticulture, or in surgery. Another appreciable requirement is that of structural simplicity, achieved thanks to the reduced number of parts and their elementary modes of interconnection.

For all the embodiments, all those construction details which are to be considered obvious for a person skilled in the art were clearly not illustrated and mentioned. Such details can in any case be subject to various design choices always within the sphere of known teaching. In particular, these details may relate to the way in which the blades and/or handles mutually engage/stop, the way the various parts overlap and their optimal shaping and dimensioning (above all in terms of ergonomics), the construction of the pivotal connections and their exact positioning, the possible elastic means for hindering the change from the open configuration to the closed one (and vice versa assisting the reverse change), the locking means for stably maintaining the closure configuration with the scissors at rest, and anything else.

Variations and/or changes may be brought to the scissors with slide cutting according to the present invention without thereby departing from the scope of the same invention as defined by the appended claims.

## Claims

1. Scissors comprising: a stem (1) integrally defining a stationary blade (3) and a stationary handle (2); a truncated handle (6) pivotally connected to said stem (1) around a first rotation pin (7) ; and a movable blade (5) fractured in relation to said truncated handle (6), the assembly of said movable blade (5) and said truncated handle (6) crossing and overlapping with said stem (1) ; a cutting line (X) being defined by the line along which cutting is completed in a position of closure of said movable blade (5), the scissors being **characterized in that** said first rotation pin (7) is off-centered in relation to said cutting line (X), on the side of said movable blade (5), and **in that** said movable blade (5) is supported by said truncated handle (6) and by said stem (1) in at least two points via respective purely pivotal connections, articulated control means (9) being connected to said movable blade (5) in correspondence to at least one of said points, for controlling the movement of said movable blade (5) in response to mutual closure between said stationary handle (2) and said truncated handle (6).

2. The scissors according to claim 1, wherein said movable blade (5) is articulated in a point to said truncated handle (6), said control means (9) comprising at least one control lever (9) articulated between said movable blade (5) and said stem (1), for letting said movable blade (5) perform an absolute rotation around said first rotation pin (7), while said control means (9) drive a movement of said movable blade (5) with a rotatory component relatively to said truncated handle (6).

3. The scissors according to claim 2, wherein said movable blade (5) is pivotally connected to said truncated handle (6) around a second rotation pin (8) located at the rear of the cutting edge (5a) of said movable blade (5), said control lever (9) being arranged between said stationary handle (2) and a tail (5b) of said movable blade (5) extending in a rearward direction beyond said second rotation pin (8).

4. The scissors according to claim 3, wherein said truncated handle (6) is substantially L-shaped, comprising a foot (6a) extending transversally and hinged at its free end, via said first rotation pin (7), to a central diagonal stem portion (4) joining said stationary handle (2) and said stationary blade (3), said movable blade (5) being pivotally connected at a corner end of said foot (6a) by means of said second rotation pin (8).

5. The scissors according to claim 4, wherein a nosepiece (5c) projects transversally from said movable blade (5) in a central position, that is to say between said cutting edge (5a) and said tail (5b), said second rotation pin being (8) arranged between said truncated handle (6) and said nosepiece (5c).

6. The scissors according to claim 2, wherein said movable blade (15) is pivotally connected to said truncated handle (16) around a second rotation pin (18) located at the rear of the cutting edge (15a) of said movable blade (15), said control lever (19) being arranged between said movable blade (15), connected in a point ahead to said second rotation pin (18), and a central diagonal stem portion (14) joining said stationary handle (12) and said stationary blade (13).

7. The scissors according to claim 6, wherein said truncated handle (16) is substantially L-shaped, having a foot (16a) developing transversally and hinged at its free end, via said first rotation pin (17), to said central stem portion (14), said movable blade (15) being pivotally connected to said foot (16a) near a corner end thereof, by means of said second rotation pin (18).

8. The scissors according to claim2, wherein said movable blade (25) is indirectly connected to said truncated handle (26) by means of an articulation arm (28) arranged at the rear of the cutting edge (25a) of said movable blade (25), said control means comprising two control levers (29', 29") arranged between said movable blade (25) and a central diagonal stem portion joining said stationary blade (23).and said stationary handle (22).

9. The scissors according to claim 8, wherein one (29') of said control levers (29', 29") extends between said central diagonal stem portion and a point in said movable blade (25) coinciding with the point of pivotal connection of said arm (28) for articulation of the movable blade itself to said truncated handle (26).

10. The scissors according to claim 2, wherein said movable blade (35') is pivotally connected to said truncated handle (36) around a second rotation pin (38') located at the rear of the cutting edge of said movable blade, the scissors also comprising a second movable blade (35") pivotally supported by said truncated handle (36) around an additional second rotation pin (38"), said control means comprising two control levers (39', 39") arranged respectively between said stem and respective movable blades (35', 35"), said control levers (39', 39") being connected to said movable blades (35', 35") at a point ahead to the relevant second rotation pin (38', 38"), for causing said movable blades (35', 35") to become mutually closed along said cutting line (X).

11. The scissors according to claim 10, wherein said truncated handle (36) has a transversally extending foot (36a), in which there are arranged, in a substantially aligned sequence: said first rotation pin (37) for the pivotal connection to a central stem portion (34) joining said stationary handle (32) and said stationary blade (33); and said couple of second rotation pins (38', 38") for the pivotal connection of said truncated handle (36) to respective movable blades (35', 35").

12. The scissors according to claim 11, wherein said stationary blade (33) comprises two arms (33', 33") extending frontally from said central stem portion (34), defining in cooperation a V-shaped cavity (33a), substantially symmetrical in relation to said cutting line (X), with a function of support and abutment of the object to be cut.

13. The scissors according to claim 1, wherein said movable blade (45) is articulated to said stem (41) around a second rotation pin (48) which is off-centered from said cutting line (X), in a greater way with respect to the extent to which said first rotation pin is off-centered, said control means comprising at least one control lever (49) articulated between said truncated handle (46) and said movable blade (45).

14. The scissors according to claim 13, wherein said control lever (49) extends between said truncated handle (46) and a tail (45b) of said movable blade (45), said tail (45b) extending in a rearward direction beyond said second rotation pin (48).

## Patentansprüche

1. Schere, enthaltend: einen Stamm (1), der integral eine stationäre Klinge (3) und einen stationären Griff (2) definiert; einen trunkierten Griff (6), der mit dem Stamm (1) um einen ersten Drehstift (7) drehbar verbunden ist; und eine bewegliche Klinge (5), die in Relation zu dem trunkierten Griff (6) gebrochen ist, wobei der Zusammenbau der beweglichen Klinge (5) und des trunkierten Griffs (6) den Stamm (1) kreuzt und überlappt; wobei eine Schneidelinie (X) durch die Linie definiert ist, längs welcher ein Schneiden in einer Schließposition der beweglichen Klinge (5) abgeschlossen ist, welche Schere **dadurch gekennzeichnet ist, dass** der erste Drehstift (7) in Relation zu der Schneidelinie (X) auf der Seite der beweglichen Klinge (5) versetzt ist, und dadurch, dass die bewegliche Klinge (5) von dem trunkierten Griff (6) und von dem Stamm (1) in wenigstens zwei Punkten durch entsprechende reine Drehverbindungen abgestützt ist, wobei artikulierte Steuereinrichtungen (9) mit der beweglichen Klinge (5) in Korrespondenz zu wenigstens einem der Punkte verbunden sind, um die Bewegung der beweglichen Klinge in Abhängigkeit von dem gegenseitigen Schließen zwischen dem stationären Griff (2) und dem trunkierten Griff (6) zu steuern.

2. Schere nach Anspruch 1, wobei die bewegliche Klinge (5) in einem Punkt zu dem trunkierten Griff (6) artikuliert ist, welche Steuereinrichtungen (9) wenigstens einen Steuerhebel (9) enthalten, der zwischen der beweglichen Klinge (5) und dem Stamm (1) artikuliert ist, um die bewegliche Klinge (5) eine absolute Drehung um den ersten Drehstift (7) ausführen zu lassen, während die Steuereinrichtungen (9) eine Bewegung der beweglichen Klinge (5) mit einer Drehkomponente relativ zu dem trunkierten Griff (6) antreiben.

3. Schere nach Anspruch 2, wobei die bewegliche Klinge (5) mit dem trunkierten Griff (6) um einen zweiten Drehstift (8) drehbar verbunden ist, der an der Rückseite der Schneidkante (5a) der beweglichen Klinge (5) liegt, wobei der Steuerhebel (9) zwischen dem stationären Griff (2) und einem Ausläufer (5b) der beweglichen Klinge (5) angeordnet ist, der in einer Rückwärtsrichtung über den zweiten Drehstift (8) hinaus verläuft.

4. Schere nach Anspruch 3, wobei der trunkierte Griff (6) im Wesentlichen L-förmig ist, einen Fuß (6a) enthält, der quer verläuft, und an seinem freien Ende über den ersten Drehstift (7) an einem zentralen diagonalen Stammteil (4) angelenkt ist, der den stationären Griff (2) und die stationäre Klinge (3) verbindet, wobei die bewegliche Klinge (5) drehbar an einem Eckende des Fußes (6a) mittels des zweiten Drehstiftes (8) angeschlossen ist.

5. Schere nach Anspruch 4, wobei ein Nasenstück (5c) quer von der beweglichen Klinge (5) in einer zentralen Position absteht, das heißt zwischen der Schneidkante (5a) und dem Ausläufer (5b), wobei der zweite Drehstift (8) zwischen dem trunkierten Griff (6) und dem Nasenstück (5c) angeordnet ist.

6. Schere nach Anspruch 2, wobei die bewegliche Klinge (15) mit dem trunkierten Griff (16) um einen zweiten Drehstift (18) drehbar verbunden ist, der an der Rückseite der Schneidkante (15a) der beweglichen Klinge (15) liegt, wobei der Steuerhebel (19) zwischen der beweglichen Klinge (15), die an einem Punkt, der vor dem zweiten Drehstift (8) liegt, angeschlossen ist, und einem zentralen diagonalen Stammteil (14) angeordnet ist, der den stationären Griff (12) und die stationäre Klinge (13) verbindet.

7. Schere nach Anspruch 6, wobei der trunkierte Griff (16) im Wesentlichen L-förmig ist, einen Fuß (16a) hat, der quer verläuft, und an seinem freien Ende über den ersten Drehstift (17) an dem zentralen Stammteil (14) angelenkt ist, wobei die bewegliche Klinge (15) drehbar mit dem Fuß (16a) nahe einer Ecke davon mittels des zweiten Drehstiftes (18) verbunden ist.

8. Schere nach Anspruch 2, wobei die bewegliche Klinge (25) indirekt mit dem trunkierten Griff (26) mittels eines Artikulationsarms (28) verbunden ist, der an der Rückseite der Schneidkante (25a) der beweglichen Klinge (25) liegt, wobei die Steuereinrichtungen zwei Steuerhebel (29', 29") enthalten, die zwischen der beweglichen Klinge (25) und einem zentralen diagonalen Stammteil angeordnet sind, der die stationäre Klinge (23) und den stationären Griff (22) verbindet.

9. Schere nach Anspruch 8, wobei einer (29') der Steuerhebel (29', 29") zwischen dem zentralen diagonalen Stammteil und einem Punkt in der beweglichen Klinge (25) verläuft, der mit dem Punkt der Drehverbindung des Arms (28) zur Artikulation der beweglichen Klinge selbst mit dem trunkierten Griff (26) zusammenfällt.

10. Schere nach Anspruch 2, wobei die bewegliche Klinge (35') mit dem trunkierten Griff (36) um einen zweiten Drehstift (38') drehbar verbunden ist, der an der Rückseite der Schneidkante der beweglichen Klinge liegt, wobei die Schere auch eine zweite bewegliche Klinge (35") enthält, die von dem trunkierten Griff (36) um einen zusätzlichen zweiten Drehstift (38") drehbar gehalten ist, wobei die Steuereinrichtungen zwei Steuerhebel (39', 39") enthalten, die entsprechend zwischen dem Stamm und jeweiligen beweglichen Klingen (35', 35") angeordnet sind, wobei die Steuerhebel (39', 39") mit den beweglichen Klingen (35', 35") an einem Punkt vor dem relevanten zweiten Drehstift (38', 38") verbunden sind, um die beiden beweglichen Klingen (35', 35") zu veranlassen, längs der Schneidelinie (X) gegenseitig geschlossen zu werden.

11. Schere nach Anspruch 10, wobei der trunkierte Griff (36) einen quer verlaufenden Fuß (36a) hat, in welchem in im Wesentlichen ausgerichteter Abfolge angeordnet sind: der erste Drehstift (37) für die Drehverbindung mit einem zentralen Stammteil (34), der den stationären Griff (32) und die stationäre Klinge (33) verbindet; und das Paar von zweiten Drehstiften (38', 38") für die Drehverbindung des trunkierten Griffs (36) mit den jeweiligen beweglichen Klingen (35', 35").

12. Schere nach Anspruch 11, wobei die stationäre Klinge (33) zwei, sich frontal von dem Stammteil (34) erstreckende Arme (33', 33") enthält, die zusammen einen V-förmigen Hohlraum (33a) im Wesentlichen symmetrisch in Relation zu der Schneidelinie (X) bilden, mit einer Funktion des Haltens und der Anlage des Gegenstandes, der zu schneiden ist.

13. Schere nach Anspruch 1, wobei die bewegliche Klinge (45) an dem Stamm (41) um einen zweiten Drehstift (48) artikuliert ist, der aus der Schneidelinie (X) in größerem Umfang bezüglich dem Ausmaß versetzt ist, mit dem der erste Drehstift versetzt ist, wobei die Steuereinrichtungen wenigstens einen Steuerhebel (49) enthalten, der zwischen dem trunkierten Griff (46) und der beweglichen Klinge (45) artikuliert ist.

14. Schere nach Anspruch 13, wobei der Steuerhebel (49) zwischen dem trunkierten Griff (46) und einem Ausläufer (45b) der beweglichen Klinge (45) verläuft, wobei der Ausläufer (45b) in einer Rückwärtsrichtung über den zweiten Drehstift (48) hinaus verläuft.

## Revendications

1. Ciseaux comprenant ; une tige (1) définissant une lame stationnaire (3) et une poignée stationnaire (2) faisant partie intégrante de la tige ; une poignée tronquée (6) montée pivotante sur ladite tige (1) autour d'une première goupille de rotation (7) ; et une lame mobile (5) libre par rapport à ladite poignée tronquée (6), l'ensemble de ladite lame mobile (5) et de ladite poignée tronquée (6) se croisant en se superposant avec ladite tige (1) ; une ligne de coupe (X) étant définie par la ligne selon laquelle une coupe est effectuée dans une position de fermeture de ladite lame mobile (5), les ciseaux étant **caractérisés en ce que** ladite première goupille de rotation (7) est excentrée par rapport à ladite ligne de coupe (X), du côté de ladite lame mobile (5), et **en ce que** ladite lame mobile (5) est supportée par ladite poignée tronquée (6) et par ladite tige (1) en au moins deux points par l'intermédiaire de connexions respectives purement pivotantes, des moyens de commande articulés (9) étant reliés à ladite lame mobile (5) en correspondance avec au moins l'un des dits points, pour commander le mouvement de ladite lame mobile (5) en réponse à une fermeture mutuelle entre ladite poignée stationnaire (2) et ladite poignée tronquée (6).

2. Ciseaux selon la revendication 1, où ladite lame mobile (5) est articulée en un point de ladite poignée tronquée (6), lesdits moyens de commande (9) comprenant au moins un levier de commande (9) articulé entre ladite lame mobile (5) et ladite tige (1), pour permettre à ladite lame mobile (5) d'effectuer une rotation absolue autour de ladite première goupille de rotation (7), tandis que lesdits moyens de commande (9) induisent un mouvement de ladite lame mobile (5) avec une composante de rotation par rapport à ladite poignée tronquée (6),

3. Ciseaux selon la revendication 2, où ladite lame mobile (5) est montée pivotante par rapport à ladite poignée tronquée (6) autour d'une seconde goupille de rotation (8) située à l'arrière du bord de coupe (5a) de ladite lame mobile (5), ledit levier de commande (9) étant agencé entre ladite poignée stationnaire (2) et une queue (5b) de ladite lame mobile (5) s'étendant vers l'arrière au-delà de ladite seconde goupille de rotation (8).

4. Ciseaux selon la revendication 3, où ladite poignée tronquée (6) est sensiblement en forme de L, comprenant un pied (6a) s'étendant transversalement et articulé à son extrémité libre, via ladite première goupille de rotation (7), sur une portion centrale en diagonale (4) de la tige reliant ladite poignée stationnaire (2) et ladite lame stationnaire (3), ladite lame mobile (5) étant montée pivotante sur une extrémité en coin du dit pied (6a) au moyen de ladite seconde goupille de rotation (8).

5. Ciseaux selon la revendication 4, où un appendice (5c) fait saillie transversalement à partir de ladite lame mobile (5) dans une position centrale, c'est-à-dire entre ledit bord de coupe (5a) et ladite queue (5b), ladite seconde goupille de rotation (8) étant agencée entre ladite poignée tronquée (6) et ledit appendice (5c).

6. Ciseaux selon la revendication 2, où ladite lame mobile (15) est montée pivotante sur ladite poignée tronquée (16) autour d'une seconde goupille de rotation (18) située à l'arrière du bord de coupe (15a) de ladite lame mobile (15), ledit levier de commande (19) étant agencé entre ladite lame mobile (15), connectée en un point à l'avant de ladite seconde goupille de rotation (18), et une portion de tige centrale en diagonale (14) reliant ladite poignée stationnaire (12) et ladite lame stationnaire (13).

7. Ciseaux selon revendication 6, où ladite poignée tronquée (16) est sensiblement en forme de L, ayant un pied (16a) se prolongeant transversalement et articulé à son extrémité libre, via ladite première goupille de rotation (17), sur ladite partie de tige centrale (14), ladite lame mobile (18) étant montée pivotante sur ledit pied (16a) à proximité d'une extrémité en coin de celui-ci, au moyen de la dite seconde goupille de rotation (18).

8. Ciseaux selon la revendication 2, où ladite lame mobile (25) est indirectement reliée à ladite poignée tronquée (26) au moyen d'un bras d'articulation (28) agencé à l'arrière du bord de coupe (25a) de ladite lame mobile (25), lesdits moyens de commande comprenant deux leviers de commande (29', 29") agencés entre ladite lame mobile (25) et une portion de tige centrale en diagonale reliant ladite lame stationnaire (23) et ladite poignée stationnaire (22).

9. Ciseaux selon la revendication 8, ou l'un (29') des dits leviers de commande (29', 29") s'étend entre ladite portion de tige centrale en diagonale et un point de ladite lame mobile (25) coïncidant avec le point de raccordement en rotation du dit bras (28) pour l'articulation de ladite poignée mobile elle-même avec ladite lame tronquée (26).

10. Ciseaux selon la revendication 2, où ladite lame mobile (35') est montée pivotante sur ladite poignée tronquée (36) autour d'une seconde goupille de rotation (38') située à l'arrière du bord de coupe de ladite lame mobile, les ciseaux comprenant en outre une seconde lame mobile (35") portée en rotation par ladite poignée tronquée (36) autour d'une seconde goupille de rotation additionnelle (38"), lesdits moyens de commande comprenant deux leviers de commande (39', 39") respectivement agencés entre ladite tige et les lames mobiles respectives (35', 35"), lesdits leviers de commande (39', 39") étant reliés aux dites lames mobiles (35', 35") en un point en avant de la seconde goupille de rotation correspondante (38', 38"), pour amener lesdites lames mobiles (35', 35") à se fermer mutuellement le long de ladite ligne de coupe (X).

11. Ciseaux selon la revendication 10, où ladite poignée tronquée (36) a un pied (36a) s'étendant transversalement, dans lequel sont agencés, selon un ordre sensiblement aligné : ladite première goupille de rotation (37) pour le montage en rotation sur une portion de tige centrale (34) reliant ladite poignée stationnaire (32) et ladite lame stationnaire (33) ; et ledit couple de secondes goupilles de rotation (38', 38") pour le montage en rotation de ladite poignée tronquée (36) par rapport aux dites lames mobiles respectives (35', 35").

12. Ciseaux selon la revendication 11, où ladite lame stationnaire (33) comprend deux bras (33', 33") s'étendant frontalement à partir de ladite portion de tige centrale (34), définissant en coopération une cavité en forme de V (33a), sensiblement symétrique par rapport à ladite ligne de coupe (X), avec une fonction de support et de butée de l'objet à couper.

13. Ciseaux selon la revendication 1, où ladite lame mobile (45) est articulée sur ladite tige (41) autour d'une seconde goupille de rotation (48) qui est excentrée par rapport à ladite ligne de coupe (X), d'une quantité plus grande que celle dont ladite première goupille de rotation est excentrée, lesdits moyens de commande comprenant au moins un levier de commande (49) articulé entre ladite poignée tronquée (46) et ladite lame mobile (45).

14. Ciseaux selon la revendication 13, où ledit levier de commande (49) s'étend entre ladite poignée tronquée (46) et une queue (45b) de ladite lame mobile (45), ladite queue (45b) s'étendant vers l'arrière au-delà de ladite seconde goupille de rotation (48).
